(19) Europäisches Patentamt
European Patent Office
Office européen des brevets

(11) Veröffentlichungsnummer: **0 417 535 A2**

(12) # EUROPÄISCHE PATENTANMELDUNG

(21) Anmeldenummer: **90116329.5**

(51) Int. Cl.$^5$: **G01N 21/64**

(22) Anmeldetag: **25.08.90**

(30) Priorität: **11.09.89 CH 3294/89**

(43) Veröffentlichungstag der Anmeldung: **20.03.91 Patentblatt 91/12**

(84) Benannte Vertragsstaaten: **BE CH DE FR GB IT LI NL**

(71) Anmelder: **F. HOFFMANN-LA ROCHE AG
Postfach 3255
CH-4002 Basel(CH)**

(72) Erfinder: **Barner, Richard, Dr.
Traubenweg 16
CH-4108 Witterswil(CH)**

Erfinder: **Huber, Walter, Dr.
Ziegelhofweg 62
CH-4303 Kaiseraugst(CH)**
Erfinder: **Hübscher, Josef
Säspelstrasse 1
CH-4208 Nunningen(CH)**
Erfinder: **Müller, Francis
Seltisbergerstrasse 18
CH-4059 Basel(CH)**

(74) Vertreter: **Kloter, Rudolf et al
Postfach 3255 Grenzacherstrasse 124
CH-4002 Basel(CH)**

(54) **Optischer Sauerstoffsensor.**

(57) Es wird ein optischer Sauerstoffsensor beschrieben, der auf einer Uebertragung elektromagnetischer Energie von einem Donor auf einen Akzeptor basiert.

Als $O_2$-Indikatoren bzw. als Donoren werden Pyrenderivate der allgemeinen Formel

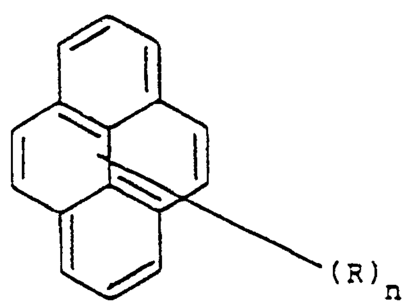

I

worin R mit einer Hydroxy-, Amino-, oder einer Gruppe der Formel -NH(1-8C Alkyl)- substituierte Alkylgruppe mit bis zu 30 C-Atomen bedeutet, eine Gruppe-$CH_2$ -OR bedeutet, wobei R die obengenannte Bedeutung hat, oder eine Gruppe -OR, bedeutet, wobei R die obengenannte Bedeutung hat, oder eine Gruppe $R'$-O-$R''$ bedeutet, wobei $R'$ eine Alkylengruppe bedeutet, $R''$ die obige Bedeutung von R hat und $R'$ und $R''$ zusammen nicht mehr als 30 C-Atome aufweisen und n eine ganze Zahl von 1-10 bedeutet, verwendet.

Bevorzugte Pyrenderivate sind 1-Hydroxymethylpyren, 1-(11-Hydroxiundecyl)-pyren, 1-(11-Hydroxiundecyloximethyl)pyren oder 1-(2-Aminoäthyloxymethyl)pyren.

Als Akzeptoren werden Perylenderivate der allgemeinen Formel

EP 0 417 535 A2

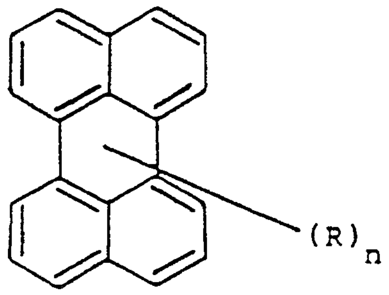

II

worin R und n die obgenannte Bedeutung haben,
verwendet.

Bevorzugte Akzeptoren sind 3-Hydroxymethylperylen, 11-Amino-1-(3-perylenmethoxi)undecan, 3-(2-Aminoäthyloxi-methyl)perylen oder 3-(11-Hydroxiundecyloxymethyl)perylen.

## OPTISCHER SAUERSTOFFSENSOR

Die vorliegende Erfindung betrifft einen optischen Sauerstoffsensor. Derartige Sensoren bestehen vielfach aus einem lichtleitenden Medium, auf das eine sauerstoffsensitive Schicht aufgebracht wurde. Solche Sensoren können einem Patienten implantiert werden und dienen insbesondere zur Überwachung des Sauerstoffgehaltes bei Intensivpatienten.

Solche optische Sauerstoff($O_2$)sensoren sind in der Patentliteratur und der Literatur beschrieben, vgl. z.B. Europäische Patentanmeldung, Veröffentlichungs-Nr. 91,390, Europäische Patentanmeldung, Veröffentlichungs-Nr. 262,578 sowie Journal of Optical Sensors, 1986, Vol. 1, No. 1, pp. 43 - 67.

Zum Beispiel kann bei diesen Sensoren Licht über eine Optik und einen dichroitischen Spiegel in eine Glasfaser (lichtleitendes Medium) eingekoppelt werden. Dies regt am Ende der Glasfaser in der $O_2$-sensitiven Schicht den $O_2$-Indikator an. Das Fluoreszenzlicht des $O_2$-Indikators wird in der Glasfaser gesammelt und durch diese wieder an den Eingang der Faser zurückgeführt. Dieses Fluoreszenzlicht wird über den dichroitischen Spiegel, Interferenzfilter und Linsen dem Photomultiplier zugeführt. Aus dem Signal des Photomultipliers werden unter zu Hilfenahme einer Auswerteelektronik Aussagen über Fluoreszenzintensität bzw. Fluoreszenzlebensdauer möglich. Die beiden Grössen können in Bezug gesetzt werden zu der $O_2$-Konzentration in der Nähe der $O_2$-sensitiven Schicht.

Das $O_2$-sensitive Element eines $pO_2$-Sensors kann auf zwei grundsätzlich verschiedene Arten ausgebildet sein.

Die $O_2$-sensitive Schicht kann auf das Faserende aufgebracht werden. Das Anregungslicht tritt aus dem Faserende aus und regt die Fluoreszenz der $O_2$-Indikatoren an. Da das abgestrahlte Fluoreszenzlicht isotrop verteilt ist, wird ein Teil wieder in die Faser eingekoppelt und durch die Faser dem Photomultiplier zugeführt.

Die sensitive Schicht kann über eine gewisse Strecke die Auskleidung der Faser ersetzen. Das Anregungslicht wird in dieser Ausbildung des sensitiven Elementes über den gesamten Bereich der sensitiven Schicht im Kern der Glasfiber geführt. Die Wechselwirkung der $O_2$-Indikatoren mit dem Anregungslicht erfolgt über das sogenannte "evanescente" Feld (Teil des elektromagnetischen Feldes, welches bei Totalreflektion in das optisch dünnere Medium eindringt). Von dieser Anregung sind nur Indikatoren betroffen, welche sich innerhalb der Reichweite dieses evanescenten Feldes befinden. Die Einkopplung des Fluoreszenzlichtes erfolgt hier über das "evanescente" Feld. Die $O_2$-sensitive Schicht besteht aus $O_2$-Indikatormolekülen. Die $O_2$-Indikatormoleküle werden in Polymeren eingebracht, welche unter dem Sammelbegriff Silikone zusammengefasst sind. Der einfachste Vertreter dieser Klasse ist Polydimethylsiloxan. Eine Vielzahl von Gruppen (z.B. Alkyl, Phenyl, Trifluoropropyl, Vinyl, Wasserstoff etc.) kann Methyl als Substituent an Silizium ersetzen. Darüberhinaus können einzelne repetierende Einheiten durch Verzweigungspunkte ersetzt sein. Diese erlauben eine Quervernetzung einzelner linearer Ketten. Die Folgen dieser Quervernetzung sind hochmolekulare Silikongummis. Silikon wird aufgrund seiner hohen $O_2$-Permeabilität bzw. $O_2$-Löslichkeit als Trägermaterial für die $O_2$-Indikatoren gewählt.

Die Darstellung der Silikonmatrix erfolgt z.B. ausgehend von sogenannten RTVs(room-temperature-vulcanizing systems) welche unter dem Einfluss der Luftfeuchtigkeit aushärten. Sie enthalten als reaktive Gruppen im allgemeinen Acyloxi-, Amin-, Oxim- oder Alkoxi-Reste. Die RTVs werden in einem Lösungsmittel gelöst, welches die $O_2$-Indikatormoleküle enthält. Diese Lösung wird in einer dünnen Schicht auf das lichtleitende Substrat aufgebracht. In Kontakt mit Umgebungsfeuchtigkeit erfolgt das Aushärten des Polymers.

Erfindungsgemäss dienen als $O_2$-Indikatoren Pyrenderivate oder eine Kombination von Pyrenderivaten und Perylenderivaten als Donor-Akzeptor-Paar (vergl. Applied Spectroscopy Vol. 42, No. 6, 1988, p. 1009-1011). Sowohl Pyrenderviate als auch die Kombination von Pyrenderivaten und Perylenderivaten bringen die spektralen Anforderungen mit, die an Fluoreszenzindikatoren für einen $O_2$-Nachweis gestellt werden. Der Vorteil der Kombination Pyrenderivat-Perylenderivat gegenüber Pyrenderivat beruht hauptsächlich in der grösseren Differenz zwischen Anregungs- und Emissionswellenlänge.

Für die "in vivo"-Anwendung eines $pO_2$-Sensors oder für das Aufbringen zusätzlicher Schichten auf der $O_2$-sensitiven Schicht (z.B. zum Aufbau eines Enzymsensors auf der Basis eines $pO_2$-Sensors) muss das Auswaschen von Indikatormolekülen verhindert werden. Im Rahmen der vorliegenden Erfindung wurde nun gefunden, dass bei geeigneter Substitution dieser Indikatormoleküle diese gleichzeitig mit der Aushärtung der Silikonmatrix an das Polymergerüst kovalent gebunden werden. Diese kovalente Bindung beruht auf der Reaktion zwischen den reaktiven Gruppen des RTV-Prepolymers sowie der reaktiven Gruppen der Indikatormoleküle.

Als $O_2$-Indikatoren bzw. als Donoren werden Pyrenderivate der allgemeinen Formel

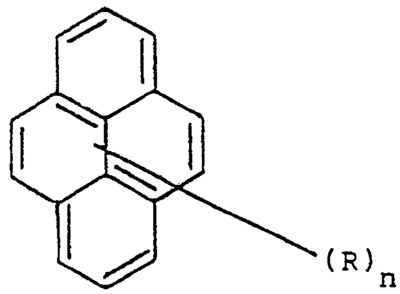

I

worin R mit einer Hydroxy-, Amino-, oder einer Gruppe der Formel -NH(1-8C Alkyl)- substituierte Alkylgruppe mit bis zu 30 C-Atomen bedeutet, eine Gruppe $-CH_2-OR$ Gruppe bedeutet, wobei R die obengenannte Bedeutung hat, oder eine Gruppe -OR, bedeutet, wobei R die obengenannte Bedeutung hat, oder eine Gruppe $R'$-O-$R''$ bedeutet, wobei $R'$ eine Alkylengruppe bedeutet, $R''$ die obige Bedeutung von R hat und $R'$ und $R''$ zusammen nicht mehr als 30 C-Atome aufweisen und n eine ganze Zahl von 1-10 bedeutet, verwendet.

Bevorzugte Pyrenderivate sind 1-Hydroxymethylpyren, 1-(11-Hydroxiundecyl)-pyren, 1-(11-Hydroxiundecyloximethyl)pyren oder 1-(2-Aminoethyloxymethyl)pyren.

Als Akzeptoren werden Perylenderivate der allgemeinen Formel

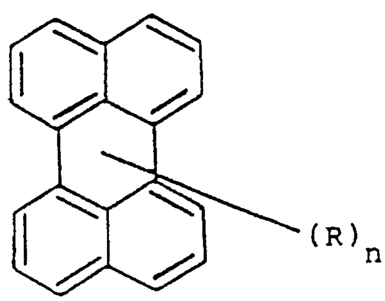

II

worin R und n die obengenannte Bedeutung haben,
verwendet.

Bevorzugte Akzeptoren sind 3-Hydroximethylperylen, 11-Amino-1-(3-perylenmethoxi)undecan, 3-(2-Aminoethyloximethyl)perylen oder 3-(11-Hydroxiundecyloximethyl)perylen.

Die Pyren- bzw. Perylenderivate liegen bevorzugt in einer Konzentration von $10^{-4}$ - $10^{-2}$ Mol/Liter vor.

Nach einem ersten Aspekt betrifft die vorliegende Anmeldung somit einen optischen Sauerstoffsensor enthaltend eine Silikonmatrix, in welcher kovalent ein Pyrenderivat der allgemeinen Formel I gebunden ist.

Nach einem weiteren Aspekt betrifft die vorliegende Erfindung einen optischen Sauerstoffsensor basierend auf einer Übertragung elektromagnetischer Energie von einem Donor auf einen Akzeptor, enthaltend eine Silikonmatrix, in welcher ein Pyrenderivat der allgemeinen Formel I als Donor und ein Perylenderivat der allgemeinen Formel II als Akzeptor kovalent gebunden ist.

Nach einem weiteren Aspekt betrifft die vorliegende Erfindung eine Silikonmatrix für eine optischen Sauerstoffsensor, in welcher kovalent ein Pyrenderivat der allgemeinen Formel I gebunden ist.

Schliesslich betrifft die vorliegende Erfindung eine Silikonmatrix für eine optischen Sauerstoffsensor basierend auf einer Übertragung elektromagnetischer Energie von einem Donor auf einen Akzeptor, in welcher ein Pyrenderivat der allgemeinen Formel I kovalent gebunden ist und als Donor dient und in welcher ein Perylenderivat der allge meinen Formel II kovalent gebunden ist und als Akzeptor dient.

Die Verbindungen der Formeln I oder II können in an sich bekannter Weise durch Alkylierung von Pyren oder Perylen mit den entsprechend substituierten Alkylhalogeniden in einer Friedel Crafts Reaktion mit einem Katalysator wie z.B. Aluminiumchlorid in einem inerten Lösungsmittel, wie Tetrachlorkohlenstoff, Methylenchlorid oder in desaktivierten flüssigen Aromaten, wie Nitrobenzol, Chlorobenzol, vorzugsweise Methylenchlorid bei 0 - 30°C, bevorzugt Raumtemperatur, hergestellt werden. Die Einführung der Alkylgruppen erfolgt entsprechend den für den betreffenden Aromaten charakteristischen Substitutionsmuster der elektrophilen Substitution. Nach üblicher Aufarbeitung mit Eiswasser und Methylenchlorid können die Alkylierungsprodukte durch Chromatographie getrennt werden.

Die Verbindungen der Formel I oder II, worin R $CH_2$-OR R′-O-R″ oder OR bedeuten, können durch Überführung der entsprechenden Hydroxiverbindung in das Alkoholat durch Deprotonierung mit Natriumhydrid bei 0 °C bis Raumtemperatur in einem inerten für die anschliessende Alkylierung günstigen Lösungsmittel wie Tetrahydrofuran oder N,N-Dialkylformamid und Umsetzung (nach abgeklungener $H_2$-Entwicklung) mit dem entsprechenden substituierten Alkylhalogenid bei Raumtemperatur in die entsprechenden substituierten Alkoxy-Verbindungen überführt werden.

Die nachfolgenden Beispiele erläutern die Erfindung:

## Beispiel 1

Es werden 2 g (10mMol) Pyren in 10 ml trockenem Methylenchlorid zu einer Suspension von 2,5 g (10mMol) 11-Bromundecanol und 20 g (160mMol) Aluminiumchlorid in 10 ml Methylenchlorid zugetropft, worauf während 16 Std. bei Raumtemperatur gerührt wird. Es wird mit Eiswasser hydrolisiert, mit Methylenchlorid extrahiert und eingeengt. Es wird mit Methylenchlorid chromatographiert. Es werden dabei 1,9 g (50%) 1-(11-Hydroxyundecyl)-pyren erhalten. HPLC (0,5% EtOAc/Hexan) $t_R$ = 3,4 Min. (Ed = 2,1min.) erhalten.

## Beispiel 2

Es werden 1,86 g (8mMol) 1-Hydroximethylpyren in 20 ml trockenem DMF gelöst und mit 300 mg (9mMol) Natriumhydrid (80% in Mineralöl) versetzt und 30 Min. bei Raumtemperatur gerührt. Dann werden 2 g (8mMol) 1-Brom-11-undecanol zugetropft und 3 Std. bei Raumtemperatur gerührt. Es wird mit Eiswasser versetzt, mit Toluol extrahiert und die organische Phase eingeengt und an Kieselgel chromatographiert. Es werden 3,4 g (99%) 1-(11-Hydroxiundecyloximethyl)pyren vom Schmelzpunkt 62-64° erhalten und im MS und NMR charakterisiert. DC (Kieselgel/Toluol) Rf (Edukt) = 0,1 Rf (Produkt) = 0,11 und HPLC (RP18 125-4/MeCN) tR (Edukt) = 2,3 Min. tR (Produkt) = 6,5min.

## Beispiel 3

Es werden 282 mg (1mMol) 3-Hydroximethylperylen in 5 ml trockenem DMF gelöst und mit 35 mg (1,2mMol) Natriumhydrid (80% in Mineralöl) versetzt und 30 Min. bei Raumtemperatur gerührt. Dann werden 0,25 g (1mMol) 1-Brom-11-undecanol zugetropft und 3 Std. bei Raumtemperatur gerührt. Es wird mit Eiswasser versetzt, mit Toluol extrahiert und die organische Phase eingeengt und an Kieselgel chromatographiert. Es werden 3,4 g (99%) 3-(11-Hydroxiundecyloximethyl)perylen vom Schmelzpunkt 126-127° erhalten und im MS und NMR charakterisiert. DC (Kieselgel/Toluol) Rf (Edukt) = 0,1 Rf (Produkt) = 0,11 und HPLC (RP18 125-4/MeCN) tR (Edukt) = 3,1 Min. tR (Produkt) = 9,5min.

## Beispiel 4

Es werden 6,20 g (27mMol) 1-Hydroximethylpyren in 10 ml trockenem DMF gelöst und mit 760 mg (27mMol) Natriumhydrid (80% in Mineralöl) versetzt und 30 Min. bei Raumtemperatur gerührt. Dann werden 16 g (52mMol) 1,11-Dibromundecan zugetropft und 16 Std. bei Raumtemperatur gerührt. Es wird mit Eiswasser versetzt, mit Toluol extrahiert und die organische Phase durch Kieselgel filtriert und eingeengt. Es wird mit Toluol an Kieselgel chromatographiert. Man erhält 8,54 g (63%) 11-Brom-1-(pyrenylmethyloxi)-undecan vom Schmelzpunkt 45-47 °C das im MS und NMR charakterisiert wird. DC (Kieselgel/Toluol) Rf (Edukte) = 0,1 und 0,9 Rf (Produkt) = 0,8 Rf (Produkt) = 0,5 und HPLC (RP18 125-4/MeCN) tR (Edukt) = 2,3 Min. tR (Produkt) = 4,6 Min.

## Beispiel 5

1 g (ca. 2mMol) 11-Brom-1-(1-Pyrenmethyloxi)undecan werden in 10 ml THF gelöst und mit 3 g Phthalimid-Kalium über Nacht bei Raumtemperatur gerührt. Es wird eingeengt und anschliessend mit Wasser und Methylenchlorid extrahiert. Nach dem Trocknen wird die Methylenchloridlösung mit 10 ml Hydrazinhydrat versetzt und über Nacht gerührt. Es wird eingeengt, in Ethanol suspendiert, filtriert und das Filtrat wird eingeengt. Man erhält 0,8 g (ca. 100%) 11-Amino-1-(1-pyrenylmethoxi)undecan. Zur Reinigung für die Elementaranalyse wird aus 1n Salzsäure umkristallisiert. Man erhält 0,5 g des entsprechenden Hydrochlorides.

## Beispiel 6

Es werden 0,90 g (2mMol) 3-(11-Hydroxiundecyloximethyl)perylen in 10 ml trockenem Pyridin gelöst und mit 500 mg (2,5mMol) Tosylchlorid versetzt und 16 Std. bei Raumtemperatur gerührt. Dann wird 1 Std. mit 10 ml Eiswasser verührt, mit Hyflo versetzt und flitriert. Der Rückstand wird erst mit Wasser gewaschen und dann mit Methylenchlorid aufgeschlämmt. Es wird mit Natriumsulfat getrocknet und eingeengt. Dabei werden 1,2 g 11-Toxyloxi-1-(3-perylenmethyloxi)undecan erhalten. Das DC auf Kieselgel mit Toluol ergab einen Rf-Wert von 0,5.

## Beispiel 7

1,2 g (ca. 2mMol) 11-Tosyloxi-1-(3-Perylenmethyloxi)undecan werden in 10 ml THF gelöst und mit 3 g Phthalimid-Kalium über Nacht bei Raumtemperatur gerührt. Es wird eingeengt und anschliessend mit Wasser und Methylenchlorid extrahiert. Nach dem Trocknen wird die Methylenchloridlösung mit 10 ml Hydrazinhydrat versetzt und über Nacht gerührt. Es wird eingeengt, in Ethanol suspendiert, filtriert und das Filtrat eingeengt. Man erhält dabei 0,9 (ca. 100%) 11-Amino-1-(3-perylenmethyloxi)undecan mit einem Rf-Wert von 0,6.

## Beispiel 8

500 mg 1-Hydroximethylpyren werden in 10 ml trockenem Dimethylformamid gelöst und bei -20° 100 mg Natriumhydrid (55% in Mineralöl) zugegeben. Es wird 30 Min. gerührt und dann 0,5 ml 2-Chlorethylamin zugetropft. Es wird über Nacht bei Raumtemperatur nachgerührt, anschliessend erst mit Ethanol und dann mit Wasser versetzt. Es wird einge engt und aus Chloroform/Hexan umgefällt. Man erhält 410 mg 1-(2-Aminoethyloximethyl)pyren.

## Beispiel 9

50 mg 3-Hydroximethylperylen werden in Analogie zu Beispiel 8 zu 3-(2-Aminoethyloximethyl)perylen umgesetzt.

## Beispiel 10 (Herstellung einer $O_2$-sensitiven Schicht)

1 g Elastosil E 43 (Wacker Chemie) wird in 1 ml einer Toluollösung des Indikators 1-(11-Hydroxiundecyloximethyl)pyren (Konzentration ca. $10^{-3}$ Mol/l) gelöst. Die resultierende dickflüssige Masse wird in gewünschter Schichtdicke auf das Glassubstrat gebracht. Nach dem Aushärten des Polymers wird nicht kovalent gebundener Indikator durch Spülen mit Methylenchlorid aus dem Polymer ausgewaschen.

Beispiel 11 [Herstellung einer $O_2$ -sensitiven Schicht)

1 g Elastosil E 43 (Wacker Chemie) wird in 1 ml einer Toluollösung von 1-(11-Hydroxiundecyloximethyl)pyren und 3-(11-Hydroxiundecyloximethyl)perylen (Konzentration ca. $10^{-3}$ Mol/l) gelöst. Die resultierende dickflüssige Masse wird in gewünschter Schichtdicke auf das Glassubstrat gebracht. Nach dem Aushärten des Polymers werden nicht kovalent gebundene Indikatoren durch Spülen mit Methylenchlorid aus dem Polymer ausgewaschen.

## Ansprüche

1. Optischer Sauerstoffsensor enthaltend eine Silikonmatrix, in welcher kovalent ein Pyrenderivat der allgemeinen Formel

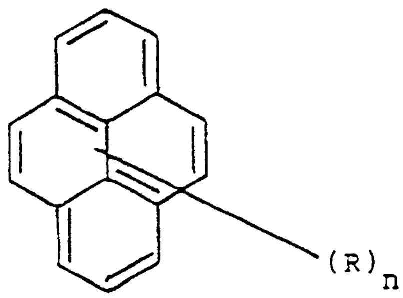

I

worin R mit einer Hydroxy-, Amino-, oder einer Gruppe der Formel -NH(1-8C Alkyl)- substituierte Alkylgruppe mit bis zu 30 C-Atomen bedeutet, eine Gruppe $-CH_2-OR$ bedeutet, wobei R die obengenannte Bedeutung hat, oder eine Gruppe -OR bedeutet, wobei R die obengenannte Bedeutung hat, oder eine Gruppe $R'-O-R''$ bedeutet, wobei $R'$ eine Alkylengruppe bedeutet, $R''$ die obige Bedeutung von R hat und $R'$ und $R''$ zusammen nicht mehr als 30 C-Atome aufweisen und n eine ganze Zahl von 1 - 10 bedeutet, gebunden ist,
wobei die Verbindung der Formel I als Sauerstoff-Indikator dient.
2. Optischer Sauerstoffsensor basierend auf einer Uebertragung elektromagnetischer Energie von einem Donor auf einen Akzeptor, enthaltend eine Silikonmatrix, in welcher ein Pyrenderivat der allgemeinen Formel I, worin R und n die obengenannte Bedeutung haben, kovalent gebunden ist und welches als Donor wirkt und weiterhin ein Perylenderivat der allgemeinen Formel

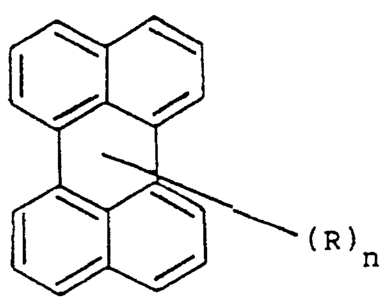

II

worin R und n die genannte Bedeutung haben,
kovalent gebunden ist und welches als Akzeptor dient.
3. Silikonmatrix für einen optischen Sauerstoffsensor, in welcher kovalent ein Pyrenderivat der allgemeinen Formel I gebunden ist und welches als $O_2$-Indikator dient.
4. Silikonmatrix für einen optischen Sauerstoffsensor basierend auf einer Uebertragung elektromagnetischer Energie von einem Donor auf einen Akzeptor, in welcher ein Pyrenderivat der allgemeinen Formel I kovalent gebunden ist und welches als Donor dient und in welcher ein Perylenderivat der allgemeinen Formel II kovalent gebunden ist und welches als Akzeptor dient.
5. Optischer Sauerstoffsensor nach Patentanspruch 1 oder 2, dadurch gekennzeichnet, dass das Pyren-

bzw. Perylenderivat in einer Konzentration von $10^{-4}$ bis $10^{-2}$ Mol/Liter vorliegt.

6. Silikonmatrix gemäss Patentanspruch 3 oder 4, dadurch gekennzeichnet, dass das Pyren- bzw. Perylenderivat in einer Konzentration von $10^{-4}$ bis $10^{-2}$ Mol/Liter vorliegt.

7. Optischer Sauerstoffsensor nach Patentanspruch 1, dadurch gekennzeichnet, dass als Sauerstoffindikator 1-Hydroxymethylpyren, 1-(11-Hydroxiundecyl)-pyren, 1-(11-Hydroxiundecyloximethyl)pyren oder 1-(2-Aminoethyloxymethyl)pyren verwendet wird.

8. Optischer Sauerstoffsensor gemäss Patentanspruch 2, dadurch gekennzeichnet, dass als Donor 1-Hydroxymethylpyren, 1-(11-Hydroxiundecyl)-pyren, 1-(11-Hydroxiundecyloximethyl)pyren oder 1-(2-Aminoethyloxymethyl)pyren verwendet wird und dass als Akzeptor 3-Hydroximethylperylen, 11-Amino-1-(3-perylenmethoxi)undecan, 3-(2-Aminoethyloximethyl)perylen oder 3-(11-Hydroxiundecyloximethyl)perylen verwendet wird.

9. Silikonmatrix gemäss Patentanspruch 3, dadurch gekennzeichnet, dass als Sauerstoffindikator 1-Hydroximethylpyren, 1-(11-Hydroxiundecyl)-pyren, 1-(11-Hydroxiundecyloximethyl)pyren oder 1-(2-Aminoethyloxymethyl)pyren verwendet wird.

10. Silikonmatrix gemäss Patentanspruch 4, dadurch gekennzeichnet, dass als Donor 1-Hydroxymethylpyren, 1-(11-Hydroxiundecyl)-pyren, 1-(11-Hydroxiundecyloximethyl)pyren oder 1-(2-Aminoethyloxymethyl)-pyren verwendet wird und dass als Akzeptor 3-Hydroximethylperylen, 11-Amino-1-(3-perylenmethoxi)-undecan, 3-(2-Aminoethyloximethyl)perylen oder 3-(11-Hydroxiundecyloximethyl)perylen verwendet wird.

11. Pyren und Perylenderivate, nämlich 1-(11-Hydroxiundecyl)-pyren, 1-(11-Hydroxiundecyloximethyl)pyren, 1-(2-Aminoethyloximethyl)pyren, 11-Amino-1-(3-perylenmethoxi)undecan, 3-(2-Aminoethyloximethyl)perylen sowie 3-(11-Hydroxiundecyloximethyl)perylen.